Europäisches Patentamt

(19) European Patent Office

Office européen des brevets

(11) Veröffentlichungsnummer : **0 028 431**
**B1**

(12) **EUROPÄISCHE PATENTSCHRIFT**

(45) Veröffentlichungstag der Patentschrift :
23.05.84

(21) Anmeldenummer : 80201007.4

(22) Anmeldetag : 23.10.80

(51) Int. Cl.³ : **A 61 B   6/02**, A 61 B   6/06

(54) **Anordnung zur Ermittlung der Streustrahlungsdichteverteilung in einem ebenen Untersuchungsbereich.**

(30) Priorität : 02.11.79 DE 2944147

(43) Veröffentlichungstag der Anmeldung :
13.05.81 Patentblatt 81/19

(45) Bekanntmachung des Hinweises auf die Patenterteilung : 23.05.84 Patentblatt 84/21

(84) Benannte Vertragsstaaten :
**BE DE FR GB IT NL SE**

(56) Entgegenhaltungen :
**DE-A- 2 461 877**
**DE-A- 2 655 230**
**FR-A- 2 232 294**
**FR-A- 2 260 977**
**FR-A- 2 383 648**
**FR-A- 2 386 055**
**FR-A- 2 405 696**
**FR-A- 2 433 926**
**US-A- 3 106 640**

(73) Patentinhaber : **Philips Patentverwaltung GmbH**
**Bilistrasse 80**
**D-2000 Hamburg 28 (DE)**
**DE**
**N.V. Philips' Gloeilampenfabrieken**
**Groenewoudseweg 1**
**NL-5621 BA Eindhoven (NL)**
**BE FR GB IT NL SE**

(72) Erfinder : **Harding, Geoffry, Dr.**
**Witt-Lönn-Strasse 44**
**D-2083 Halstenbek (DE)**
Erfinder : **Wagner, Wolfgang**
**Bookholtswiete 11**
**D-2000 Hamburg (DE)**

(74) Vertreter : **Hartmann, Heinrich, Dipl.-Ing. et al**
**Philips Patentverwaltung GmbH Bilistrasse 80 Post-**
**fach 10 51 49**
**D-2000 Hamburg 28 (DE)**

**Beschreibung**

Die Erfindung betrifft eine Anordnung zur Ermittlung der Streustrahlungsdichteverteilung in einem ebenen Untersuchungsbereich, mit wenigstens einer Gamma- oder Röntgenstrahlenquelle zur Erzeugung eines durch den Untersuchungsbereich hindurchtretenden Primärstrahls mit geringem Querschnitt, einer außerhalb des Primärstrahls angeordneten, einen Teil der durch den Primärstrahl im Untersuchungsbereich erzeugten Streustrahlung erfassenden Detektoranordnung, einer zwischen dem Untersuchungsbereich und den Detektoranordnung angeordneten Blendenanordnung, die so ausgebildet ist, daß die Detektoranordnung von dem jeweils im Primärstrahl befindlichen Teil des Untersuchungsbereiches einen Satz von von der Streustrahlungsdichte in diesem Teil abhängiger Meßwerte erzeugt, und mit einer Antriebseinrichtung zur Bewegung des Primärstrahls in bezug auf den Untersuchungsbereich. Eine solche Anordnung ist aus der DE-OS 27 13 581 bekannt.

Die Blendenanordnung wird dabei durch eine Schlitzblende mit einer schlitzförmigen Öffnung gebildet, deren Hauptausdehnungsrichtung sich in einer zum Primärstrahl ungefähr senkrechten Richtung erstreckt. Die Detektoranordnung besteht aus mehreren hinter der Blendenanordnung angeordneten Detektoren. Jeder Detektor kann nur durch die in einem bestimmten Abschnitt des Primärstrahls erzeugte Streustrahlung getroffen werden, so daß jedem Detektor ein anderer Abschnitt zugeordnet ist und alle Detektoren zusammen den durch den Untersuchungsbereich verlaufenden Teil des Primärstrahls erfassen. Die Erfindung ist aber auch bei Anordnungen anwendbar, bei denen die Blendenanordnung aus einer Vielkanalblende besteht, wie in der DE-OS 26 55 230 beschrieben. Statt einer aus vielen Detektoren bestehenden Detektoranordnung kann aber auch eine andere ortsempfindliche Nachweisvorrichtung verwendet werden, z. B. eine Gammakamera oder ein Röntgenbildverstärker. Wichtig ist nur, daß eine eindeutige räumliche Zuordnung zwischen Bereichen aus dem Primärstrahl einerseits und Bereichen aus der Detektoranordnung andererseits besteht.

In einer bestimmten Stellung des Primärstrahls relativ zum Untersuchungsbereich bzw. zu dem darin angeordneten zu untersuchenden Körper, kann jeweils nur die Streustrahlendichte in dem vom Primärstrahl getroffenen Bereich gemessen werden. Zur Ermittlung der Streudichteverteilung in einer Ebene ist es daher erforderlich, den Primärstrahl relativ zum Untersuchungsbereich zu verschieben. Diese Verschiebung erfolgt bei allen bekannten Anordnungen der eingangs genannten Art senkrecht zum Primärstrahl.

Die Erfindung basiert auf folgenden Überlegungen:

Bei den bekannten Anordnungen wird einerseits die Primärstrahlung und andererseits die in dem Körper erzeugte Streustrahlung durch Photoabsorption oder Comptonstreuung geschwächt. Diese Schwächung ist um so stärker, je länger der Weg ist, den der Primärstrahl bis zu dem Punkt, von dem die zu messende Streustrahlung ausgeht, zurücklegen muß, und je länger der Weg ist, den die in diesem Punkt erzeugte Streustrahlung durch den Körper zurückzulegen hat. Im allgemeinen ist also die die Detektoranordnung erreichende Streustrahlung am geringsten, wenn sie aus dem Zentrum des zu untersuchenden Körpers kommt. Je geringer aber die von der Detektoranordnung gemessene Strahlungsintensität ist, um so größer ist die Ungenauigkeit, mit der die so gemessenen Werte behaftet sind, so daß die Streudichteverteilung im Zentrum des zu untersuchenden Körpers nur relativ ungenau rekonstruiert werden kann. Oft liegen aber gerade die diagnostisch interessanten Bereiche im Zentrum des Körpers.

Um die Genauigkeit zu verbessern, könnte zwar die Intensität des Primärstrahls erhöht werden. Dadurch würde die dem Patienten zugeführte Strahlungsdosis jedoch erhöht werden — und zwar in allen Bereichen des Körpers im gleichen Maße —, ohne daß die Genauigkeit in den äußeren Bereichen des Körpers, wo die Primärstrahlung und/oder die Streustrahlung nur wenig geschwächt wird, wesentlich gesteigert wird. Man kann nun die Genauigkeit der für das Zentrum ermittelten Meßwerte auch dadurch verbessern, daß man den Primärstrahl — ohne seine Intensität zu erhöhen — aus verschiedenen Richtungen durch das Zentrum treten läßt und die im Überschneidungsbereich der Strahlen ermittelten Meßwerte summiert und den so gebildeten Summenwert mit einem Faktor multipliziert, der der Belegungsdichte in diesem Bereich proportional ist, was auf eine Mittelung der für diesen Bereich gemessenen Meßwerte hinausläuft. Dabei wird zwar neben der Genauigkeit auch die Dosis im Zentrum des Körpers erhöht, jedoch wird die Dosis in den Außenbereichen des Körpers praktisch nicht vergrößert.

Aufbauend auf diesen Überlegungen ist es daher Aufgabe der vorliegenden Erfindung, eine Anordnung der eingangs genannten Art so auszugestalten, daß das Zentrum des Untersuchungsbereiches öfter vom Primärstrahl erfaßt wird als die Außenbereiche.

Diese Aufgabe wird dadurch gelöst, daß die Strahlenquelle und die Blendenanordnung auf einer mit einer den Untersuchungsbereich freilassenden Öffnungen versehenen Trägeranordnung befestigt sind, die um eine den Primärstrahl schneidende und zu diesem senkrechte Achse drehbar ist.

Bei der Erfindung wird also ein kreisförmiger Untersuchungsbereich mit Hilfe eines auf sein Zentrum (die Drehachse der Trägeranordnung) gerichteten Primärstrahls abgetastet. Bei der Drehung des Trägerkörpers wird der Strahler auf einem Kreisbogen um das Zentrum des Untersuchungsbereiches gedreht, so daß sich — läßt man einmal die Schwächung der Strahlung im untersuchten Körper außer acht — eine zum Zentrum hin zunehmende Belegungsdichte ergibt. Zusammen mit der Intensitätsschwächung wird dann eine annähernd gleichmäßige Belegungsdichte erzielt. Da die Genauigkeit, mit welcher ein Streukoeffizient errechnet werden kann, von der gemessenen Anzahl der in diesem Punkt gestreuten Photonen abhängt, liefert somit die erfindungsgemäße Anordnung eine deutlich bessere Bildqualität im Zentrum der Untersuchungsschicht als die eingangs erwähnten bekannten Anordnungen, bei denen eine Verschiebung immer senkrecht zur Richtung des Primärstrahls erfolgt.

An dieser Stelle sei darauf hingewiesen, daß in der DE-A-28 31 311 bereits eine Vorrichtung zur Ermittlung innerer Körperstrukturen mittels Streustrahlung beschrieben ist, die an sich dem sogenannten Transmissions-Computertomographen entspricht, bei der jedoch zusätzlich auch noch die längs des ausgeblendeten Primärstrahls erzeugte Streustrahlung gemessen wird mittels zweier beiderseits der Untersuchungsebene angeordneter, den untersuchten Körper umgreifender Hohlzylinderdetektoren. — Die für die Messung der Streustrahlung vorgesehenen Hohlzylinderdetektoren können jedoch nur immer die im gesamten Untersuchungsbereich durch den Primärstrahl erzeugte Streustrahlung messen und nicht die Streustrahlung, die an einzelnen von dem Primärstrahl getroffenen Punkten erzeugt wird. Außerdem erfolgt dabei eine Verschiebung des Primärstrahls senkrecht zu seiner Richtung und erst, wenn der gesamte Untersuchungsbereich auf diese Weise abgetastet ist, wird die Anordnung um ein kleines Winkelinkrement gedreht. Sieht man einmal von der Intensitätsschwächung durch den zu untersuchenden Körper ab, dann ist die Belegungsdichte des Untersuchungsbereiches mit Primärstrahlung homogen — im Gegensatz zur Erfindung, wo die Belegungsdichte im Zentrum höher ist.

Eine Weiterbildung der Erfindung sieht vor, daß die Detektoranordnung ebenfalls auf der Trägeranordnung befestigt ist. Hierbei mißt jedes Element der Detektoranordnung in allen Stellungen der Trägeranordnung stets nur die aus einem bestimmten Bereich des Primärstrahls stammende Streustrahlung. Aufgrund dieser eindeutigen räumlichen Zuordnung, und aufgrund der Tatsache, daß dieser Bereich bei der Drehung des Trägerkörpers einen Kreis um die Drehachse beschreibt, mißt jeder Detektor die Streudichteverteilung in einem Kreisbogen um die Drehachse.

Eine dazu alternative Weiterbildung der Erfindung sieht vor, daß die Detektoranordnung einen feststehenden, zur Drehachse der Trägeranordnung konzentrischen Kreisbogen bildet. Zwischen dieser und der vorgenannten Ausführungsform besteht ein Unterschied, der analog zu dem Unterschied zwischen einem Transmissions-Computertomographen der vierten und der dritten Generation ist. Die Detektoranordnung benötigt im allgemeinen eine größere Anzahl von Detektorelementen als die vorgenannte Ausführungsform. Dafür ändert sich die räumliche Zuordnung zwischen einem Detektorelement und dem Primärstrahl mit der Drehung der Trägeranordnung, so daß die in einem bestimmten Bereich des Primärstrahls erzeugte Streustrahlung in jeder Drehstellung der Trägeranordnung von einem anderen Element der Detektoranordnung gemessen wird. Empfindlichkeitsunterschiede der einzelnen Elemente der Detektoranordnung haben daher auf die Rekonstruktion der Streudichteverteilung einen geringeren Einfluß ; ringförmige Artefakte, wie sie bei der zuerst genannten Ausführungsform auftreten können, wenn die Empfindlichkeit eines Detektorelementes von der Empfindlichkeit der anderen Detektorelemente abweicht, werden hierbei nicht beobachtet.

Die Genauigkeit und die Ansprechempfindlichkeit läßt sich noch dadurch steigern, daß beiderseits des Primärstrahls mehrere Blendenanordnungen vorgesehen sind, durch die hindurch je ein Element der Detektoranordnungen die in einem bestimmten Bereich des Primärstrahls erzeugte Streustrahlung erfaßt. Wenn beispielsweise zwei Blendenanordnungen auf jeder Seite des Primärstrahls vorgesehen sind, wird die in einem bestimmten Bereich des Primärstrahls erzeugte Streustrahlung von insgesamt vier Elementen der Detektoranordnung gemessen. Die vier Meßwerte werden zur Erzeugung eines der Streudichte in diesem Bereich entsprechenden Signals addiert.

Bekanntlich erlauben Anordnungen der eingangs genannten Art, also auch die erfindungsgemäße, eine einfachere Rekonstruktion der Dichteverteilung in der Ebene als sie bei der sogenannten Transmissions-Computertomographie möglich ist, weil jeder Detektor schon die in einem bestimmten Bereich bzw. Punkt erzeugte Dichte mißt und nicht etwa — wie bei der Transmissions-Computertomographie — das Linienintegral der Streudichte über den jeweiligen Strahlenpfad hinweg. Auf der anderen Seite erlaubt die rekonstruierte Streudichteverteilung nur eine qualitative Aussage, wenn nicht die Schwächung des Primärstrahls bis zu dem Punkt, in dem die Streustrahlung erzeugt wird, und die Schwächung der Streustrahlung auf ihrem Weg zum jeweiligen Detektorelement berücksichtigt wird. Grundsätzlich kann dies mit Hilfe eines geeigneten Rechenprogramms erfolgen, in dem bestimmte Annahmen über die mittlere Streudichte und die Ausdehnung des zu untersuchenden Objektes getroffen sind. Naturgemäß können die damit erhaltenen Dichteverteilungen, die sich noch durch geeignete Iterationsverfahren verbessern lassen, aufgrund dieser Annahmen im allgemeinen nicht exakt sein.

Eine genauere Ermittlung der Streustrahlungsdichteverteilung ist nach einer Weiterbildung der Erfindung dadurch möglich, daß ein Röntgenstrahler vorgesehen ist, dessen Strahlung durch einen Kollimator derart ausblendbar ist, daß seine Strahlung den gesamten Untersuchungsbereich durchsetzt und von einer hinter dem Untersuchungsbereich angeordneten Detektoranordnung erfaßt wird. Der Röntgenstrahler bildet hierbei zusammen mit der erwähnten Detektoranordnung einen Teil eines Computertomographen der dritten oder vierten Generation (je nachdem, ob die Detektoranordnung mitbewegt wird oder nicht), der mittels eines Rechners die Absorptionsverteilung in der Untersuchungsebene bestimmen kann. Der zusätzliche Aufwand hierfür ist gering, weil die Detektoranordnung und die Mechanik zum Drehen des Strahlers um den Untersuchungsbereich (Trägeranordnung) ohnehin schon vorhanden sind.

Aus der damit ermittelten Absorptionsdichteverteilung läßt sich für jeden einzelnen Meßwert dessen Schwächung errechnen, da der Weg des Primärstrahls bis zum Streupunkt und vom Streupunkt zum jeweiligen Detektorelement festliegen. Es müssen dann lediglich die längs dieses Weges ermittelten Schwächungskoeffizienten addiert werden, so daß die Gesamtschwächung errechnet werden kann. Die von den einzelnen Detektorelementen gemessenen Werte müssen dann mit einem Gewichtungsfaktor gewichtet werden, der dem so für den gesamten Strahlenweg errechneten Schwächungsfaktor proportional ist. — Die dem Patienten bei dieser Bestimmung der Absorptionsverteilung verabfolgte Dosis kann wesentlich geringer gehalten werden als bei Transmissions-Computertomographen. Die in den einzelnen Punkten ermittelten Absorptionskoeffizienten sind dabei zwar weniger genau, doch schlagen diese Rekonstruktionsfehler nicht voll durch, weil zur Bestimmung der Schwächung eines Primär- bzw. Streustrahles auf seinem Weg durch den Untersuchungsbereich die Schwächungskoeffizienten vieler Punkte addiert werden müssen, wobei Fehler wenigstens teilweise wieder herausgemittelt werden. Es kommt hinzu, daß dabei zweckmäßigerweise der Röntgenstrahler mit der gleichen hohen Spannung (z. B. 350 kV) betrieben werden kann, wie bei der Ermittlung der Streudichteverteilung, so daß die Dosis auch aufgrund dieser Tatsache verringert wird.

Auf der anderen Seite ist es aber auch möglich, für die Erstellung der Absorptionsverteilung den Röntgenstrahler mit der gleichen Röhrenspannung und der gleichen Intensität zu betreiben wie bei der Transmissions-Computertomographie üblich. Aufgrund der so ermittelten Absorptionsverteilung läßt sich dann die Streudichteverteilung genau rekonstruieren. Anhand der so ermittelten Streudichteverteilungen wiederum können die Streuanteile im Transmissions-Computertomogramm (die Absorption in einem einzelnen Punkt des Transmissions-Computertomogramms setzt sich aus einem Schwächungsanteil durch Photoabsorption und einem Schwächungsanteil durch Streuung zusammen) bestimmt und von den dort ermittelten Werten subtrahiert werden, woraus sich die Photoabsorption ergibt. Am Schluß dieser Berechnungen liegen dann zwei Dichteverteilungen vor. Einerseits die Streudichteverteilung im Untersuchungsbereich und andererseits die Photoabsorptionsdichteverteilung im Untersuchungsbereich. Daraus kann der Mediziner zusätzliche diagnostische Informationen gewinnen.

Die Erfindung wird nachstehend anhand der Zeichnung näher erläutert. Es zeigen

Figur 1 eine erste Ausführungsform der Erfindung mit zwei einander gegenüber angeordneten Röntgenstrahlern und auf der Trägeranordnung befestigten Detektoranordnungen,

Figur 2 eine entsprechende Anordnung, die jedoch eine verbesserte Nachweiswahrscheinlichkeit für die Streustrahlung aufweist,

Figur 3a eine Anordnung, mit der gleichzeitig ein Transmissions-Computertomogramm angefertigt werden kann,

Figur 3b dieselbe Anordnung während des für das Transmissions-Computertomogramm erforderlichen Meßvorganges,

Figur 4 eine Anordnung, bei der die Elemente der Detektoranordnung fest auf einem Kreisbogen um die Drehachse angeordnet sind,

Figur 5a eine Fig. 4 entsprechende Anordnung, die jedoch zusätzlich auch zum Anfertigen von Transmissions-Computertomogrammen geeignet ist,

Figur 5b eine verschiebbare Kollimatorplatte und

Figur 6 ein Blockschaltbild zur Rekonstruktion der Streudichteverteilung anhand der gewonnenen Meßwerte.

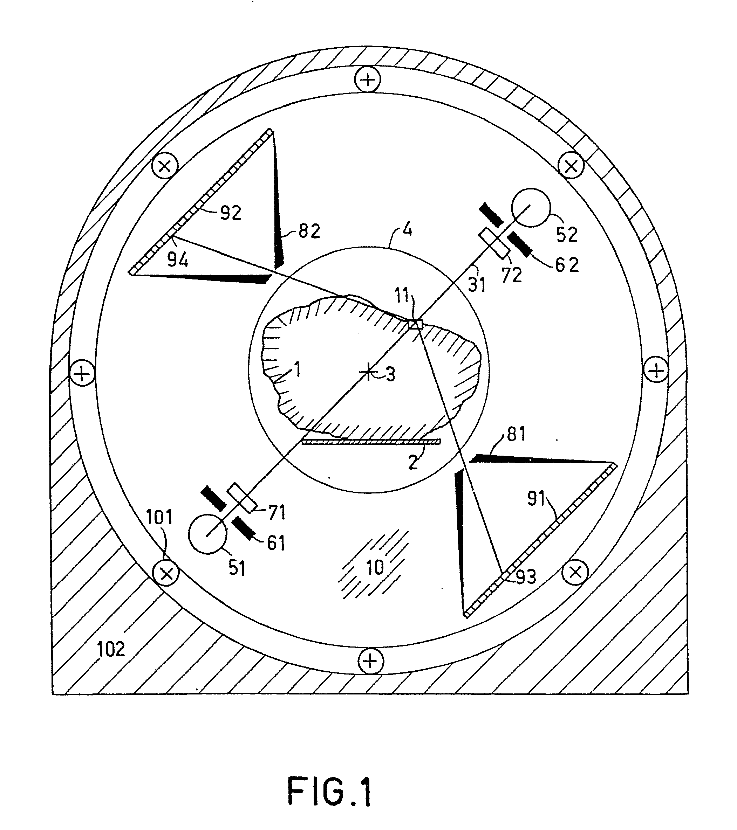

In Fig. 1 ist mit 102 ein ortsfestes Gehäuse bezeichnet, in dem mittels Rollenlagern 101 eine Trägeranordnung 10 um eine Achse 3 drehbar gelagert ist. Die Antriebseinheiten zur Drehung der Trägeranordnung 10 sind nicht näher dargestellt. Im Innern der Trägeranordnung befindet sich eine Öffnung 4, die den Untersuchungsbereich definiert, innerhalb dessen die Streustrahlung ermittelt werden kann. In diesem Untersuchungsbereich liegt auf einer Tischplatte 2 der zu untersuchende Patientenkörper 1, von dem die Streudichteverteilung in einem bestimmten Querschnitt ermittelt werden soll. Zu diesem Zweck sind beiderseits des zur Drehachse 3 konzentrisch angeordneten Untersuchungsbereiches Röntgenstrahler 51 und 52 angeordnet, vor denen sich Kollimatoren 61 und 62 befinden, die sich deckende, die Drehachse schneidende Primärstrahlen 31 ausblenden.

Für jeden der beiden Strahler ist ein Transmissionsdetektor 71 und 72 angeordnet, der mit nicht näher dargestellten Löchern versehen ist, um den Primärstrahl dieses Strahlers ungehindert passieren zu lassen, während der durch den Untersuchungsbereich geschwächte Primärstrahl des jeweils gegenüberliegenden Strahlers aufgrund der Strahlausweitung innerhalb des Körpers nachgewiesen werden kann. Mit Hilfe dieser Detektoren können etwaige Annahmen über den Schwächungsverlauf innerhalb des Körpers korrigiert werden, wie an sich in der DE-OS 27 13 581, Seite 13 bzw. 15, beschrieben.

Beiderseits des Primärstrahls 31 und jenseits des Untersuchungsbereiches 4 sind auf der drehbaren Trägeranordnung 10 zwei Detektoranordnungen 91 und 92 angebracht, die aus einer Vielzahl von nebeneinander angeordneten Detektorelementen bestehen, die in Richtung senkrecht zur Zeichenebene eine größere Ausdehnung haben, wie an sich in der DE-OS 27 13 581 beschrieben ist. Zwischen den beiden Detektoranordnungen 91 und 92 und dem Untersuchungsbereich 4 befindet sich je eine schlitzförmige Blende 81 und 82, die eine eindeutige räumliche Zuordnung zwischen einem Punkt bzw. Bereich — z. B. dem Bereich 11 — auf dem Primärstrahl 31 und der Detektoranordnung 91 und 92 herstellt, so daß die in diesem Punkt 11 des Primärstrahls erzeugte Streustrahlung von den an den Orten 93 und 94 befindlichen Elementen der Detektoranordnung gemessen wird. Die im Primärstrahl innerhalb des Untersuchungsbereiches 4 an anderen Orten erzeugte Streustrahlung wird von anderen Elementen der Detektoranordnung 91 und 92 gemessen — ähnlich wie bei der Anordnung nach der DE-OS 27 13 581.

Während bei letzter jedoch zur Erfassung der Streudichteverteilung in einer Ebene der Primärstrahl relativ zum Untersuchungsbereich senkrecht zu seiner Richtung verschoben wird, wird hier die gesamte zu untersuchende Fläche dadurch erfaßt, daß die Trägeranordnung 10 zunächst um ein kleines Winkelinkrement gedreht wird, wonach die beiden Detektoranordnungen neue Sätze von Meßwerten aufnehmen, wonach eine weitere Drehung um ein kleines Winkelinkrement erfolgt usw., bis die Anordnung um insgesamt 180° gedreht ist. Die Winkelinkremente sind dabei so gewählt, daß auch der gesamte Rand vollständig von den verschiedenen Primärstrahlen erfaßt wird. Der Primärstrahl bleibt dabei stets auf die Drehachse 3 ausgerichtet.

Es ist auch möglich, mit nur einer einzigen Detektor- und Blendenanordnung zu arbeiten ; dann wird allerdings die Nachweiswahrscheinlichkeit geringer, wenn nicht die Detektorflächen vergrößert werden. Ebenso kann anstelle zweier Röntgenstrahler nur ein einziger verwendet werden. Dann ist aber für eine vollständige Messung eine Drehung der Trägeranordnung um 360° erforderlich, damit jeder Punkt auf dem Rand des Untersuchungsbereiches 4 einmal von dem ungeschwächten Primärstrahl getroffen wird.

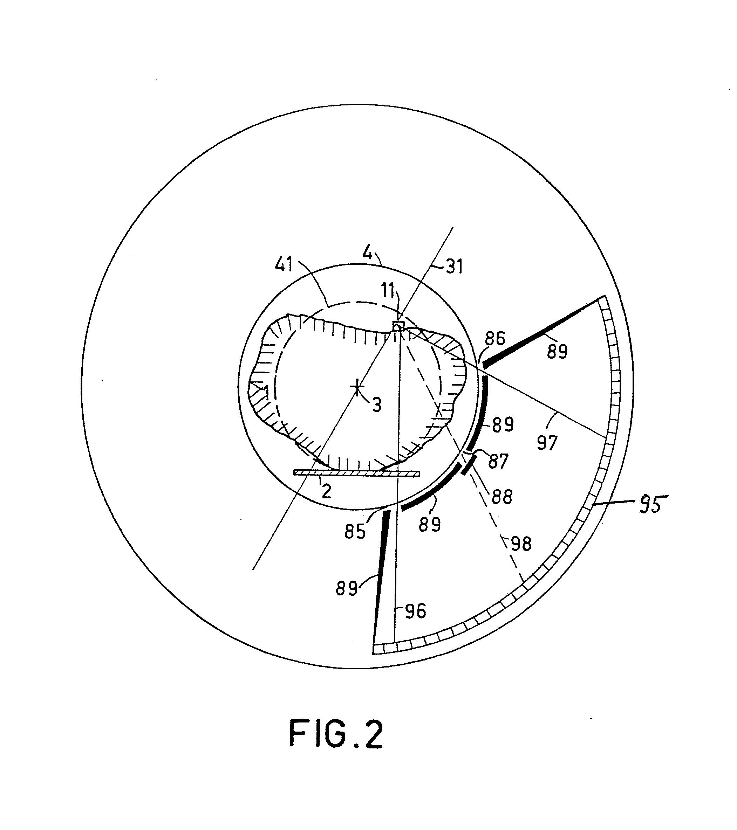

In Fig. 2 ist ein Teil einer weiteren Ausführungsform dargestellt, wobei beiderseits des Primärstrahls 31 je zwei Detektorsysteme angeordnet sind. Aus Vereinfachungsgründen sind die beiden Detektorsysteme links vom Primärstrahl 31 sowie die beiden ihn erzeugenden Röntgenstrahler nicht dargestellt. Die beiden Detektorsysteme sind zweckmäßigerweise zu einer einzigen Detektoranordnung 95 zusammengefaßt, und es ist eine Blendenanordnung 89 mit zwei Schlitzen 85 und 86 zwischen jeder Detektoranordnung und dem Untersuchungsbereich angeordnet. Die Schlitze sind so bemessen und angeordnet, daß der untere Teil der Elemente der Detektoranordnung von Streustrahlung getroffen werden kann, die im Primärstrahl 31 erzeugt wird und durch den Schlitz 85 tritt, während der obere Teil der Elemente der Detektoranordnung von Streustrahlung getroffen werden kann, die auf dem Primärstrahl innerhalb des Untersuchungsbereiches erzeugt wird und durch den Schlitz 86 tritt. Kein Element der Detektoranordnung kann dabei Strahlung messen, die sowohl durch den Schlitz 86 als auch durch den Schlitz 85 tritt und von dem Primärstrahl innerhalb des Untersuchungsbereiches 4 herrührt.

Diese Ausbildung der Detektor- und Blendenanordnung erhöht die Nachweiswahrscheinlichkeit und verbessert damit die Rekonstruktionsgenauigkeit, weil die Streustrahlung eines jeden Punktes auf dem Primärstrahl 31 von zwei verschiedenen Detektorelementen erfaßt werden können, wie in Fig. 2 für die vom Punkt 11 auf dem Primärstrahl 31 ausgehenden Streustrahlen 96 und 97, die durch die Schlitze 85 und 86 treten, angedeutet ist. Außerdem ergänzen sich beide Blenden- und Detektoranordnungen insofern als die aus der linken unteren Hälfte des Primärstrahls in Fig. 2 stammende Streustrahlung besser von dem Schlitz 85 zugeordneten Detektorelementen erfaßt wird, während Streustrahlung aus der oberen rechten Hälfte des Primärstrahls 31 besser von dem Schlitz 86 zugeordneten Elementen der Detektoranordnung erfaßt wird. Dies deshalb, weil die in der linken unteren Hälfte des Primärstrahls 31 erzeugte Streustrahlung weniger geschwächt ist, wenn sie durch den Schlitz 85 tritt, während die in der rechten oberen Hälfte des Primärstrahls erzeugte Streustrahlung weniger geschwächt ist, wenn sie durch den oberen Schlitz 86 tritt ; beispielsweise wird der Streustrahl 97, der im Punkt 11 auf der rechten oberen Hälfte des Primärstrahls 31 erzeugt wird und durch den Schlitz 86 tritt, durch den Körper 1 weniger geschwächt als der im selben Punkt erzeugte und durch den Schlitz 85 tretende Streustrahl 96.

In Fig. 2 ist die Blendenanordnung 89 mit einem weiteren Schlitz 87 versehen, der aber im Normalbetrieb durch eine Abschirmung 88 abgedeckt wird. Wenn der Querschnitt des Körpers 1 so klein ist, daß er innerhalb des gestrichelten, zur Drehachse 3 konzentrischen Kreises 41, dessen Durchmesser kleiner ist als der Durchmesser des Untersuchungsbereiches 4, Platz findet, dann kann mittels einer nicht näher dargestellten Antriebseinrichtung die Abschirmung 88 aus dem Strahlengang geschoben werden. Damit wird ein weiterer Streustrahlenweg 98 freigegeben, was die Nachweiswahrscheinlichkeit für Streustrahlung und damit die Meßgeschwindigkeit erhöht. Der Kreis 41 ist so bemessen, daß jedes Element der Detektoranordnung durch die Schlitze 85, 86 und 87 hindurch immer nur einen einzigen Punkt auf dem Primärstrahl innerhalb des Kreises 41 « sehen » kann, so daß störende Überlagerungen von Streustrahlung, die ein Detektorelement auf wenigstens zwei verschiedenen Streustrahlenwegen erreichen kann, nicht möglich sind.

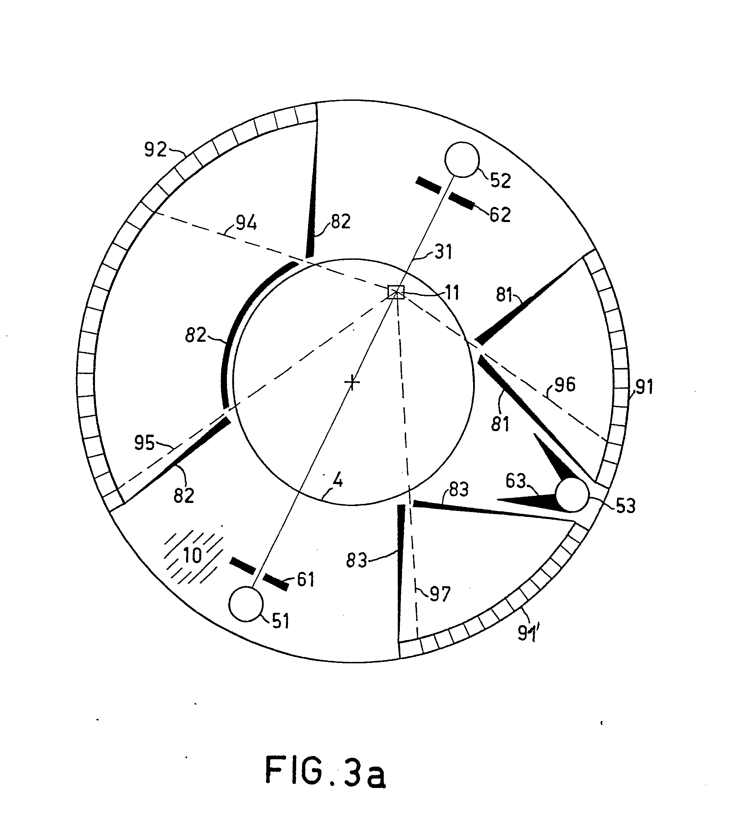

Fig. 3a zeigt ebenfalls eine Anordnung mit zwei auf der Trägeranordnung 10 angeordneten Röntgenstrahlern 51 und 52 mit davor angeordneten Kollimatoren 61 und 62, durch die der Primärstrahl 31 tritt. Beiderseits des Primärstrahls sind wiederum je zwei Detektoranordnungen angeordnet. Während jedoch auf der linken Hälfte die Elemente der beiden Detektoranordnungen 92 unmittelbar aneinander angrenzen und von einer gemeinsamen Blendenanordnung 82 mit zwei Schlitzen abgedeckt werden, sind auf der rechten Seite die beiden Detektoranordnungen 91 und 91' voneinander getrennt und mit je einer Schlitzblendenanordnung 81 und 83 versehen. Die beispielsweise im Punkt 11 erzeugte Streustrahlung erreicht längs der Linien 94, 95, 96 und 97 je ein Element in den verschiedenen Detektoranordnungen. Zwischen den beiden Detektor- bzw. Blendenanordnungen 81, 91 und 83, 91' ist ein Röntgenstrahler 53 angeordnet, der somit der Detektoranordnung 92 gegenüberliegt. Davor befindet sich ein Kollimator 63, der so ausgebildet ist, daß von dem Strahler 53 erzeugte Strahlung den Untersuchungsbereich 4 in derselben Ebene durchsetzt wie der Primärstrahl 31.

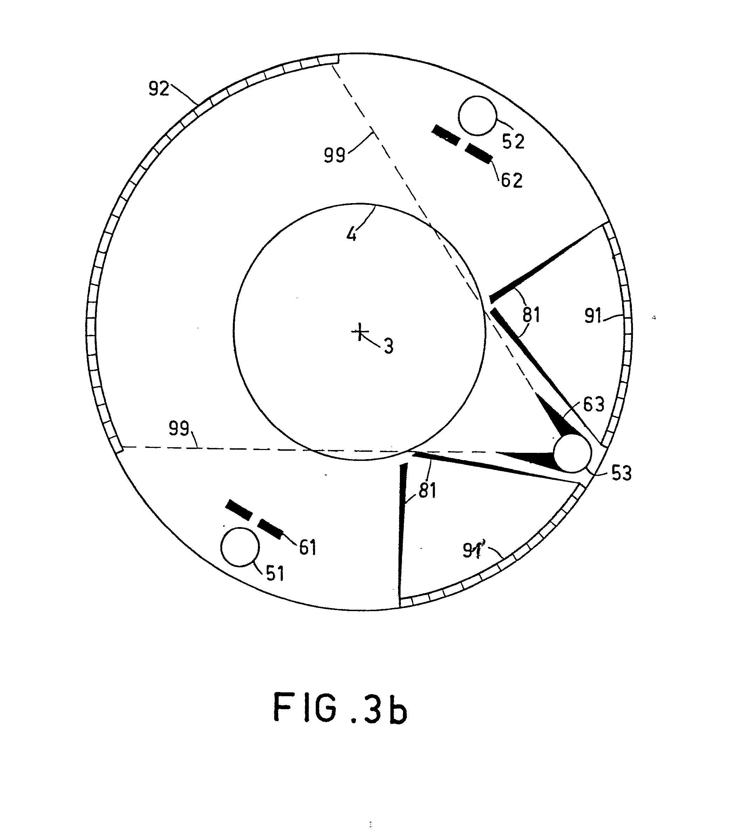

Während Fig. 3a die Anordnung in einer Untersuchungsphase zeigt, bei der der Primärstrahl 31 den Untersuchungsbereich 4 durchsetzt, um die Streudichteverteilung zu ermitteln, ist in Fig. 3b die Phase dargestellt, in der die vom Strahler 53 erzeugte Röntgenstrahlung den gesamten Untersuchungsbereich zwischen den Strahlen 99 durchsetzt, um die Schwächungsverteilung innerhalb der Untersuchungsebene 4 zu ermitteln. In dieser Phase ist durch eine nicht näher dargestellte Antriebs- und Verschiebeeinrichtung die Schlitzblendenanordnung 82 senkrecht zur Untersuchungsebene verschoben, so daß die Elemente der Detektoranordnung 92 das Strahlenbündel der Strahlenquelle 53 erfassen können.

Die beiden in Fig. 3a und 3b dargestellten Untersuchungsphasen können unmittelbar aufeinander folgen, wobei die Röhrenspannung, die zunächst während mindestens einer halben Umdrehung an die Röntgenstrahler 51 und 53 angelegt wird, danach auf den Röntgenstrahler 53 umgeschaltet wird, bis mit Hilfe der Detektoranordnung die Schwächungsverteilung innerhalb des Untersuchungsbereiches 4 vollständig erfaßt ist.

Man erkennt, daß der Aufwand für die zusätzliche Durchführung einer Transmissions-Computertomographie relativ gering ist, weil die Detektoranordnung 92 zur Erfassung der Transmissions-Meßwerte ohnehin schon für die Streustrahlungsmessung erforderlich ist und die drehbare Trägeranordnung auch bei dieser benötigt wird.

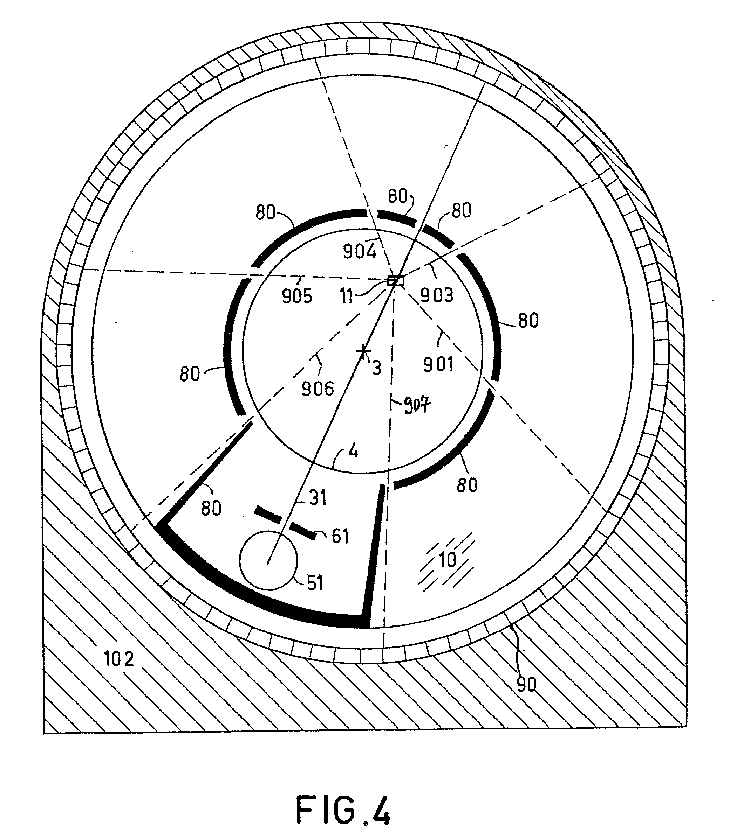

In Fig. 4 ist eine Ausführungsform dargestellt, die sich von den bisher beschriebenen dadurch unterscheidet, daß die Detektoranordnung 90 als mit dem Gehäuse 102 verbundener und daher feststehender, die Drehachse 3 und die Trägeranordnung 10 konzentrisch umschließender Kreis aus einzelnen Detektorelementen ausgebildet ist. Lediglich der Röntgenstrahler 51 mit dem Kollimator 61 zur Ausblendung des Primärstrahls 31 und die mit insgesamt sieben Schlitzen versehene Blendenanordnung 80 sind noch auf der Trägeranordnung 10 angebracht, die bei dieser Ausführungsform mit nur einem Röntgenstrahler um 360° gedreht werden muß. Der Primärstrahl 31 tritt durch einen dem Röntgenstrahler 51 diametral gegenüberliegenden Schlitz, so daß das dahinter jeweils befindliche Element der Detektoranordnung die Schwächung des Primärstrahles 31 messen kann. Die sechs anderen Schlitze in der Blendenanordnung 80 sind beiderseits des Primärstrahls so verteilt, daß in jeder Winkelstellung der Trägeranordnung 10 jedes Element der Detektoranordnung nur durch einen Schlitz von Streustrahlung getroffen werden kann, die innerhalb des Untersuchungsbereiches 4 und im Primärstrahl 31 erzeugt wird. In Fig. 4 sind wiederum die sechs Strahlenpfade 901, 903, 904, 905, 906, 907 eingezeichnet, längs derer die im Punkt 11 erzeugte Streustrahlung sechs verschiedene Elemente der Detektoranordnung erreicht.

Die in Fig. 4 dargestellte Ausführungsform benötigt in der Regel zwar mehr Detektorelemente als die vorgenannten Ausführungsformen, doch müssen diese nicht mitbewegt werden. Außerdem besteht keine feste Zuordnung zwischen einem Detektorelement und einem Kreisbogen um die Drehachse 3 ; dies bedeutet einerseits, daß ringförmige Artefakte, die bei den vorher beschriebenen Anordnungen auftreten können, wenn die Empfindlichkeit eines Detektorelementes von der der anderen abweicht, nicht auftreten können, andererseits aber auch, daß zur Ermittlung der Streudichteverteilung auf einem Kreisbogen um die Drehachse 3 die Meßwerte einer Vielzahl verschiedener Detektorelemente herangezogen werden müssen. — Außerdem müssen die in Fig. 4 nicht näher dargestellten Lagerungselemente für die Trägeranordnung 10 so angeordnet sein, daß sie die Streustrahlenpfade zu den Detektorelementen nicht verdecken.

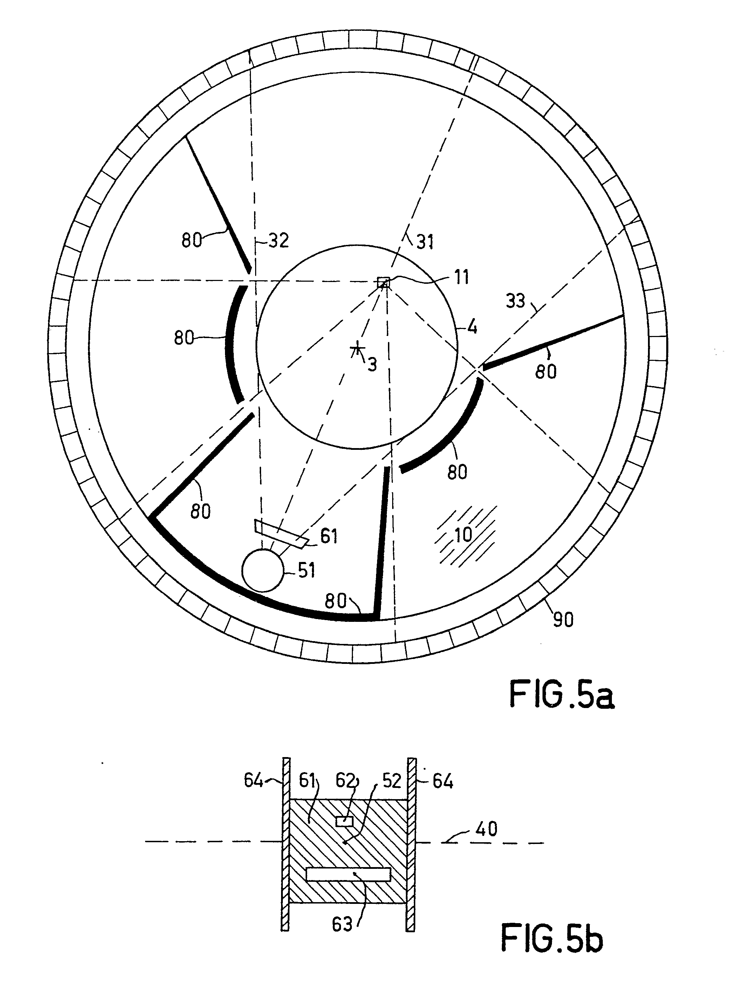

In Fig. 5a ist eine Ausführungsform mit feststehenden Detektorelementen dargestellt, die auch die Erfassung von Transmissions-Meßwerten gestattet. Die aus einem feststehenden, zur Drehachse 3 konzentrischen Ring aus Detektorelementen bestehende Detektoranordnung 90 ist ebenso wie bei Fig. 4 auch an dem nicht näher dargestellten Gestell der Anordnung befestigt. Die Blendenanordnung 80 ist ähnlich wie in Fig. 2 ausgebildet derart, daß das zwischen den Grenzstrahlen 32 und 33 den gesamten Untersuchungsbereich 4 erfassende Strahlenbündel durch die Blendenanordnung 80 nicht beeinflußt wird.

Vor dem Röntgenstrahler 51 ist eine senkrecht zur Zeichenebene verschiebbare Kollimatorplatte mit zwei senkrecht übereinander angeordneten Öffnungen 62 und 63 angeordnet (vgl. Fig. 5b). Wenn die Öffnung 62 in die Untersuchungsebene 40 geschoben ist, wird ein eng kollimierter Primärstrahl 31 ausgeblendet und es kann die Streudichteverteilung gemessen werden. Wird hingegen die Öffnung 63 in die Untersuchungsebene 40 geschoben, dann wird ein fächerförmiges Strahlenbündel mit den Grenzstrahlen 32 und 33 ausgeblendet und es können Transmissionsmessungen durchgeführt werden. Die Messungen können nacheinander durchgeführt werden, d. h. es wird während einer Drehung von 360° zunächst die Streudichteverteilung ermittelt und danach die Schwächungsverteilung — oder umgekehrt. Es ist aber auch möglich, während des Betriebes, d. h. während der Drehung der Trägeranordnung 10, die Blende 61 entlang der fest montierten zur Zeichenebene der Fig. 5a senkrechten Schienen 64 in schneller Folge hin und her zu schieben, so daß abwechselnd der Primärstrahl 31 und das Strahlenbündel mit den Randstrahlen 32 und 33 emittiert wird. Dadurch ist gewährleistet, daß die Streustrahlung und die Transmissionsstrahlung in benachbarten Drehstellungen der Trägeranordnung 10 ohne wesentliche zeitliche Verzögerung gemessen werden können.

Bei der in Fig. 5a dargestellten Anordnung besteht der zusätzliche Aufwand für die Durchführung der Computer-Tomographie einzig in der senkrecht zur Untersuchungsebene verschiebbaren Kollimatorplatte 61.

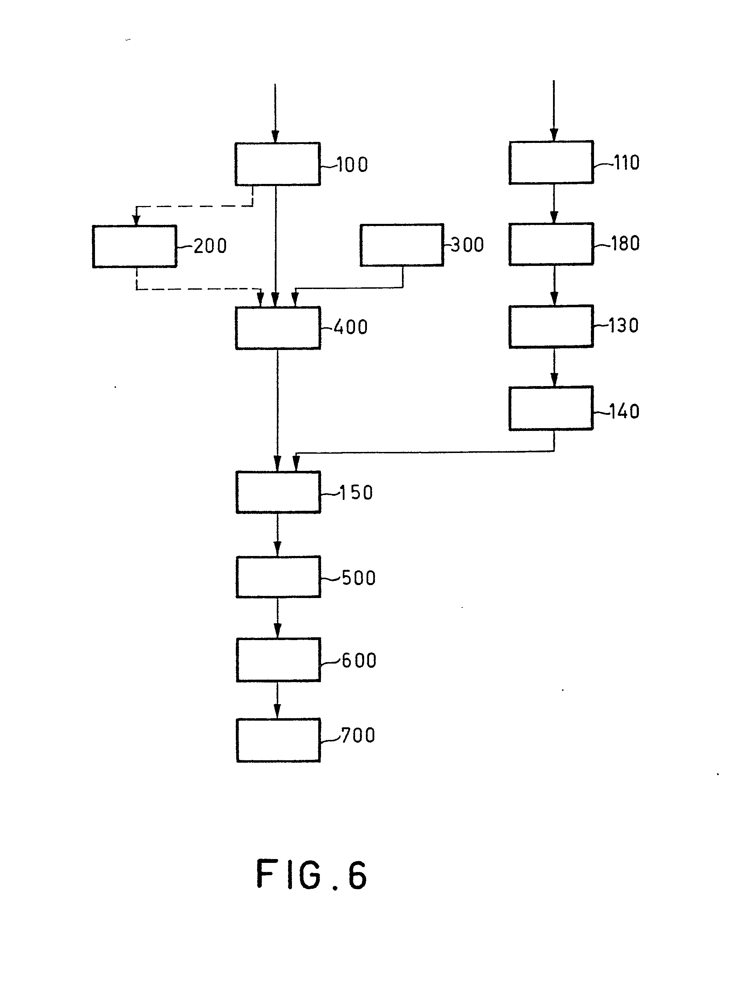

Das in Fig. 6 dargestellte Blockschaltbild zur Rekonstruktion der Streudichteverteilung aus den mit einer Anordnung nach den Fig. 3a, 3b oder 5a, 5b gemessenen Meßwerten umfaßt einen Speicher 100, dem die Streustrahlenmeßwerte zugeführt werden. Diese Meßwerte werden in der Recheneinheit 400 mit verschiedenen Gewichtungsfaktoren gewichtet. Dadurch soll berücksichtigt werden, daß für einen Punkt im Zentrum des Untersuchungsbereiches wesentlich mehr Meßwerte vorliegen als für einen Punkt außerhalb des Zentrums, so daß die Meßwerte für einen Punkt im Zentrum vor oder nach ihrer Summierung mit einem entsprechend niedrigeren Faktor gewichtet werden müssen.

Unterstellt man beispielsweise, daß die Zahl z der Abtastrichtungen und die Abmessungen des Primärstrahls so gewählt sind, daß die Primärstrahlen gerade jede Stelle auf dem äußeren Rand des Untersuchungsbereiches 4 einmal erfassen, und geht man weiter davon aus, daß die Streudichte in einem Polarkoordinatensystem in Punkten rekonstruiert werden soll, die auf Geraden durch das Zentrum liegen, deren Winkelstellungen denjenigen des Primärstrahls in bezug auf den Untersuchungsbereich entsprechen, wobei der Abstand a der Zentren zweier auf einer Geraden benachbarter Punkte der Breite des Primärstrahls entspricht, dann ergeben sich die Gewichtungsfaktoren wie folgt :

Die Meßwerte für den Punkt im Zentrum bzw. deren Summe werden mit einem Gewichtungsfaktor 1/z gewichtet, weil der Primärstrahl in allen z Winkelstellungen diesen Punkt durchsetzt. Für einen Punkt außerhalb des Zentrums werden die Meßwerte bzw. deren Summe mit einem Faktor n/N gewichtet, wobei $N = z/2\ \pi$ und n ein ganzzahliger Wert ist, der angibt, wievielmal größer der Abstand zwischen dem Zentrum 3 des Untersuchungsbereiches und dem Zentrum des betreffenden Punktes ist als der Abstand a zwischen zwei benachbarten Punkten.

Die Gewichtungsfaktorverteilung ergibt sich hierbei für ein Polarkoordinatensystem. Gegebenenfalls können daraus aber auch nach bekannten Transformationsregeln die Gewichtungsfaktoren für Punkte in einem kartesischen Koordinatensystem errechnet werden.

Zugleich mit diesem Gewichtungsfaktor können andere von der Geometrie der Anordnung abhängige Gewichtungsfaktoren, z. B. die Winkelabhängigkeit der Streustrahlung, und die unterschiedlichen Detektorempfindlichkeiten einbezogen werden. Die daraus resultierenden Gewichtungsfaktoren sind in dem Speicher 300 gespeichert.

Mit 200 ist eine Resortierungseinheit bezeichnet, welche für den Fall von stationär angeordneten Detektoren (Fig. 5a) die Meßwerte von festgespeicherten Koordinatentransformationen derart umsortiert, als wären die Meßwerte von mitgedrehten Detektorelementen (wie bei Fig. 3a) gemessen worden. In einer weiteren Recheneinheit 150 werden die so gewichteten Werte mit einem Faktor gewichtet, der der Schwächung der Strahlung durch den Körper entspricht. Je größer diese Schwächung ist, um so größer ist der Gewichtungsfaktor.

Diese Werte werden aus dem Transmissions-Computertomogramm entnommen. Die Transmissions-Meßwerte werden zu diesem Zweck im Speicher 110 zwischengespeichert, woraus im Rechner 180 die Schwächungsdichteverteilung im Untersuchungsbereich rekonstruiert wird. Für jeden einzelnen Punkt im Untersuchungsbereich läßt sich dann errechnen, wie stark die Primärstrahlung geschwächt worden ist, um bis zu diesem Punkt zu gelangen, und wie stark die Streustrahlung geschwächt worden ist, um von diesem Punkt zu einem bestimmten Detektorelement zu gelangen. Der Schwächungskoeffizient ist das Linienintegral über die Schwächungskoeffizienten entlang des Primärstrahls bis zu dem jeweiligen Punkt und von dort aus über den jeweiligen Streustrahlenpfad bis zum Detektorelement. Diese werden in einem Punkt in einer Recheneinheit 130 berechnet und im Speicher 140 zwischengespeichert und zur Gewichtung der von der Recheneinheit 400 gelieferten Werte in der Recheneinheit 150 benutzt. Aus den von der Recheneinheit 150 gelieferten Werten wird in der Rekonstruktionseinheit 500 die Streudichteverteilung berechnet, wobei je einem Element der als mitgedreht angenommenen Detektoranordnung ein konzentrischer Kreis in der Rekonstruktionsebene zugeordnet wird und je ein Meßwert auf einen Punkt dieses Kreises unter einem Polarwinkel übertragen wird, welcher dem Drehwinkel entspricht, unter dem dieser Meßwert gewonnen wurde. Nach Abschluß dieser Berechnungen wird das Bild im Speicher 600 gespeichert und auf einem Bildsichtgerät 700 dargestellt.

Anstatt durch Messung mit Hilfe eines Transmissions-Computertomogramms können die der jeweiligen Schwächung entsprechenden Gewichtungsfaktoren auch mit Hilfe von Schwächungsmessungen ermittelt werden, die an einem geeigneten Phantom durchgeführt wurden. Für verschiedene Körperquerschnitte von unterschiedlich dicken Patienten müssen dabei unterschiedliche Phantome verwendet werden, denen im Speicher 140 jeweils ein Satz von Gewichtungsfaktoren zuzuordnen ist. Hiermit ist allerdings nur eine näherungsweise Bestimmung des jeweiligen Gewichtungsfaktors möglich, die um so besser ist, je mehr der gewählte Phantomquerschnitt dem untersuchten Körperquerschnitt entspricht.

Grundsätzlich ist es auch möglich, die Schwächung der Streustrahlung bzw. die diese Schwächung berücksichtigenden Gewichtungsfaktoren durch Rechnung zu ermitteln. Dazu wird zunächst die Berandung des untersuchten Körperquerschnitts ermittelt, indem jeder Meßwert mit einem Schwellwert verglichen wird, der ungefähr dem Streukoeffizienten von Wasser entspricht oder etwas niedriger ist. Ist ein Meßwert kleiner als der Schwellwert, dann wird er dem außerhalb des Körpers liegenden Teil des Untersuchungsbereiches (Luft) zugeordnet, ist er größer, wird er dem Körperquerschnitt zugeordnet.

Dem so ermittelten Querschnitt der Körpers wird zunächst eine homogene Streudichteverteilung zugeordnet, wobei die Streudichte gleich derjenigen von Wasser angenommen wird. Dies ist eine brauchbare Näherung für die tatsächlichen Verhältnisse, weil auch der menschliche Körper zu einem großen Teil Wasser enthält. Es wird damit die Schwächung des Primärstrahls, die sich bei der angegebenen Streudichteverteilung für die einzelnen Abtastrichtungen ergibt, errechnet und mit den Schwächungswerten verglichen, die sich aus den Meßwerten der Elemente 71 und 72 (Fig. 1) ergeben. Die Differenz wird herangezogen, um die angenommene Streudichteverteilung zu korrigieren.

Aus der derart korrigierten Streudichteverteilung wird die Strahlschwächung entlang des Primärstrahlweges zum Streupunkt (z. B. 11, Fig. 2) und von diesem entlang des Streustrahlweges (z. B. 97) zum Detektor ermittelt. Diese Berechnung wird nacheinander für alle Abtastrichtungen bzw. Drehstellungen und alle Detektorpositionen durchgeführt und deren Ergebnisse werden gespeichert. Mit Hilfe der gespeicherten Strahlschwächungswerte, die jeweils die Kehrwerte der Gewichtungsfaktoren sind, werden die gemessenen Streustrahlenmeßwerte korrigiert, woraus eine verbesserte Streudichteverteilung ermittelt werden kann.

Eine weitere Verbesserung der ermittelten Streudichteverteilung kann gegebenenfalls dadurch erreicht werden, daß die für die einzelnen Punkte ermittelten Streudichtewerte erneut mit dem Streukoeffizienten von Wasser verglichen werden, um so nicht nur wie im vorher beschriebenen Vergleichszyklus; die Berandung des Körpers, sondern auch Bereiche im Innern des Körpers zu ermitteln, deren Streukoeffizient wesentlich von demjenigen von Wasser abweicht (z. B. Luft oder Knochen). Es wird danach der bereits vorher beschriebene Zyklus erneut durchlaufen, was sich gegebenenfalls wiederholen kann.

## Ansprüche

1. Anordnung zur Ermittlung der Streustrahldichteverteilung in einem ebenen Untersuchungsbereich, mit wenigstens einer Gamma- oder Röntgenstrahlenquelle zur Erzeugung eines durch den Untersuchungsbereich hindurchtretenden Primärstrahls mit geringem Querschnitt, einer außerhalb des Primärstrahls angeordneten, einen Teil der durch den Primärstrahl im Untersuchungsbereich erzeugten Streustrahlung erfassenden Detektoranordnung, einer zwischen dem Untersuchungsbereich und der Detektoranordnung angeordneten Blendenanordnung, die so ausgebildet ist, daß die Detektoranordnung von dem jeweils im Primärstrahl befindlichen Teil des Untersuchungsbereiches einen Satz von von der Streustrahlungsdichte in diesem Teil abhängiger Meßwerte erzeugt, und mit einer Antriebseinrichtung zur Bewegung des Primärstrahls in bezug auf den Untersuchungsbereich, dadurch gekennzeichnet, daß die Strahlenquelle und die Blendenanordnung auf einer mit einer den Untersuchungsbereich freilassenden Öffnung versehenen Trägeranordnung befestigt sind, die um eine den Primärstrahl schneidende und zu diesem senkrechte Achse drehbar ist.

2. Anordnung nach Anspruch 1, dadurch gekennzeichnet, daß die Detektoranordnung ebenfalls auf der Trägeranordnung befestigt ist.

3. Anordnung nach Anspruch 1, dadurch gekennzeichnet, daß die Detektoranordnung einen feststehenden, zur Drehachse der Trägeranordnung konzentrischen Kreisbogen bildet.

4. Anordnung nach einem der vorhergehenden Ansprüche, dadurch gekennzeichnet, daß beiderseits des Primärstrahls mehrere Blendenanordnungen vorgesehen sind, durch die hindurch jeweils ein Element der Detektoranordnungen die in einem bestimmten Bereich des Primärstrahls erzeugte Streustrahlung erfaßt.

5. Anordnung nach einem der vorhergehenden Ansprüche, dadurch gekennzeichnet, daß ein Röntgenstrahler vorgesehen ist, dessen Strahlung durch einen Kollimator derart ausblendbar ist, daß seine Strahlung den gesamten Untersuchungsbereich durchsetzt und von einer hinter dem Untersuchungsbereich angeordneten Detektoranordnung erfaßt wird.

6. Anordnung nach Anspruch 3 und Anspruch 5, dadurch gekennzeichnet, daß vor dem Strahler eine senkrecht zum Untersuchungsbereich verschiebbare, in zwei Stellungen arretierbare mit zwei Öffnungen versehene Kollimatoranordnung vorgesehen ist, wobei in der einen Stellung durch die eine Öffnung der Primärstrahl und in der anderen Stellung durch die andere Öffnung ein den gesamten Untersuchungsbereich erfassendes Strahlenbündel ausgeblendet wird.

## Claims

1. An arrangement for determining the scattered ray density distribution in a plane examination region, comprising at least one gamma radiation or X-ray source for generating a narrow primary beam which passes through the examination region, a detector device which is arranged outside the primary beam and which intercepts a part of the scattered radiation produced by the primary beam in the examination region, a diaphragm device which is arranged between the examination region and the detector device and which is constructed so that for the portion of the examination region each time situated within the primary beam the detector device produces a set of measurement values which are dependent on the scattered radiation density in said portion and also comprising a drive device for moving the primary beam with respect to the examination region, characterized in that the radiation source and the diaphragm device are mounted on a supporting device which leaves the examination region free and which is rotatable about an axis which intersects the primary beam and which is directed perpendicularly thereto.

2. An arrangement as claimed in Claim 1, characterized in that the detector device is also mounted on the supporting device.

3. An arrangement as claimed in Claim 1, characterized in that the detector device is in the form of the stationary arc of a circle which is concentric with respect to the axis of rotation of the supporting device.

4. An arrangement as claimed in any one of the preceding Claims, characterized in that on both sides of the primary beam there are arranged several diaphragm devices through which each time an element of the detector devices intercepts the scattered radiation produced in a given zone of the primary beam.

5. An arrangement as claimed in any one of the preceding Claims, characterized in that there is provided an X-ray source whose radiation can be stopped by a collimator so that its radiation passes through the entire measuring area and is intercepted by a detector device which is arranged behind the examination region.

6. An arrangement as claimed in Claim 3 and Claim 5, characterized in that in front of the radiation source there is arranged a collimator device which is displaceable perpendicularly to the examination region, which can be locked in two positions and which comprises two apertures, one aperture in one position defining the primary beam and the other aperture in the other position defining a radiation beam which covers the entire examination region.

## Revendications

1. Dispositif pour mesurer la répartition de densité d'un rayonnement dispersé dans une zone plane à examiner, comportant au moins une source de rayons X ou de rayons gamma destinée à produire un faisceau primaire de faible section traversant la zone à examiner, un dispositif de détection monté à l'extérieur du faisceau primaire et détectant une fraction du rayonnement dispersé produit par le faisceau primaire dans la zone à examiner, un dispositif à diaphragme monté entre la zone à examiner et le dispositif de détection et agencé de telle sorte que le dispositif de détection produise, à partir de la partie de la zone à examiner se trouvant dans le faisceau primaire, une série de valeurs de mesure dépendant de la densité du rayonnement dispersé dans cette partie, et un dispositif d'entraînement pour déplacer le faisceau primaire par rapport à la zone à examiner, caractérisé en ce que la source de rayons et le dispositif à diaphragme sont fixés sur un dispositif de support pourvu d'une ouverture dégageant la zone à examiner, ce dispositif de support pouvant tourner autour d'un axe intersectant le faisceau primaire et perpendiculaire à celui-ci.

2. Dispositif suivant la revendication 1, caractérisé en ce que le dispositif de détection est également fixé sur le dispositif de support.

3. Dispositif suivant la revendication 1, caractérisé en ce que le dispositif de détection forme un arc de cercle fixe concentrique à l'axe de rotation du dispositif de support.

4. Dispositif suivant l'une quelconque des revendications précédentes, caractérisé en ce que plusieurs dispositifs à diaphragme sont prévus de part et d'autre du faisceau primaire à travers lesquels un élément des dispositifs de détection détecte chaque fois le rayonnement dispersé produit dans une zone déterminée du faisceau primaire.

5. Dispositif suivant l'une quelconque des revendications précédentes, caractérisé en ce qu'une source de rayons X est prévue, le rayonnement de cette source pouvant être diaphragmé par un collimateur d'une manière telle qu'il traverse l'ensemble de la zone à examiner et qu'il soit détecté par un dispositif de détection monté derrière la zone à examiner.

6. Dispositif suivant les revendications 3 et 5, caractérisé en ce qu'un dispositif collimateur pourvu de deux ouvertures, pouvant être déplacé perpendiculairement à la zone à examiner et pouvant être arrêté dans deux positions est prévu, étant entendu que, dans la première position, le faisceau primaire est diaphragmé par la première ouverture et que, dans l'autre position, un faisceau de rayons couvrant toute la zone à examiner est diaphragmé par l'autre ouverture.

FIG.1

FIG.2

FIG.3a

FIG.3b

FIG.4

FIG.5a

FIG.5b

FIG.6